# EUROPEAN PATENT APPLICATION

(11) **EP 3 808 362 A1**
(43) Date of publication of application: **21.04.2021**
(21) Application number: 19810753.4
(22) Date of filing: 21.05.2019
(51) Int. Cl.: A61K 38/08, A61K 38/10, A61K 38/16, A61P 17/18, A61P 39/06, C07K 7/06

(54) **REACTIVE OXYGEN PRODUCTION INHIBITOR AND/OR SCAVENGING PROMOTER**

(30) Priority: 31.05.2018 JP 2018105652
(71) Applicant: National University Corporation Okayama University, Kita-ku Okayama-shi Okayama 700-8530 (JP)
(72) Inventor: NISHIBORI, Masahiro, Okayama-shi, Okayama 700-8530 (JP); MORI, Shuji, Okayama-shi, Okayama 700-8530 (JP); WAKE, Hidenori, Okayama-shi, Okayama 700-8530 (JP)
(74) Representative: Hertin und Partner Rechts- und Patentanwälte PartG mbB
(86) International application number: PCT/JP2019/020181
(87) International publication number: WO 2019/230509

(57) **Abstract**

The present invention provides a production inhibitor and/or scavenging promoter for reactive oxygen, and relates to a production inhibitor and/or scavenging promoter for reactive oxygen containing as an active ingredient a peptide having histidine-rich sequence. The histidine-rich sequence is the amino acid sequence identified by any one or a plurality of the following SEQ ID NOs: 2 to 8; (SEQ ID NO: 2) DLHPH; (SEQ ID NO: 3) KHHSH; (SEQ ID NO: 4) EQHPH; (SEQ ID NO: 5) GHHPH; (SEQ ID NO: 6) AHHPH; (SEQ ID NO: 7) EHDTH; and (SEQ ID NO: 8) RQHPH.

## Description

### Technical Field

The present invention relates to a production inhibitor and/or scavenging promoter for reactive oxygen. Specifically, the present invention relates to a production inhibitor and/or scavenging promoter for reactive oxygen containing as an active ingredient a peptide having a histidine-rich sequence.

The present application claims priority from Japanese Patent Application No. 2018-105652, which is incorporated herein by reference.

### Background Art

Reactive oxygen species (ROS) include radical and non-radical species. Of the reactive oxygen species in a broad sense including lipid-related substances, examples of the radical species include a hydroxy radical (·OH), an alkoxy radical (LO·), a peroxy radical (LOO·), a hydroperoxy radical (HOO·), nitrogen monoxide (NO·), nitrogen dioxide (NO₂·), and a superoxide anion radical (O₂⁻·) in the order from the radical having the highest reactivity. Examples of the non-radical species include singlet oxygen (¹O₂), ozone (O₃), hydrogen peroxide (H₂O₂), and lipid hydroperoxide (LOOH).

Excess *in vivo* reactive oxygen species are removed by actions of *in vivo* antioxidant mechanisms, such as superoxide dismutase (SOD), catalase, and glutathione peroxidase. However, when the antioxidant mechanisms are degraded by aging, disease, and the like, various disease risks due to the reactive oxygen species are increased. For example, when iron is accumulated in the liver through excessive intake of iron, generation of hydroxy radicals is promoted by Fenton's reaction, resulting in an onset of hepatitis or the like. As a Fenton's reaction inhibitor, there is a disclosure of a pharmaceutical agent containing D-cysteinolic acid as an active ingredient (Patent Literature 1). In Patent Literature 1, there is a description that D-cysteinolic acid is used to inhibit production of hydroxy radicals or scavenge singlet oxygen. There is a disclosure that astaxanthin and curcumin are used in a reactive oxygen inhibitor (PatentLiterature2). In Patent Literature 2, there is a description that astaxanthin and curcumin are used as a reactive oxygen inhibitor for preventing oxidation of a component that is liable to be oxidized, such as a lipid, in a food or a pharmaceutical. However, the pharmaceutical agents described in Patent Literatures 1 and 2 each have a problem in that enteric absorption thereof is extremely low, resulting in low bioavailability. Immediately after being generated, a hydroxy radical reacts with a nearby molecule to exhibit a potent oxidizing power. In addition, the hydroxy radical induces a reversible or irreversible cell damage, and conceivably as a result of this, various diseases are caused. In order to prevent such cell damage, disease, and the like, many pharmaceutical agents are being developed. However, the hydroxy radical has an extremely high reactivity and an extremely short lifetime, and hence a pharmaceutical agent that traps the hydroxy radical after generation of the hydroxy radical is limited in effect.

Histidine-rich glycoprotein (HRG) is a plasma protein having a molecular weight of about 80 kDa, which was identified by Heimburger et al. (1972). HRG is a histidine-rich protein formed of a total of 507 amino acids including 66 histidines, is mainly synthesized in the liver, and is present in human plasma at a concentration of from about 100 µg/mL to about 150 µg/mL, which is regarded as extremely high. It is known that HRG is involved in regulation of coagulation and fibrinolytic systems and regulation of angiogenesis (Non Patent Literature 1). Further, there are disclosures of: methods for the inhibition of angiogenesis by administering a polypeptide containing a part of HRG (hereinafter sometimes referred to as "HRG polypeptide (s) ") ; and pharmaceutical compositions and articles of manufacture including HRG polypeptides, antibodies and receptors that bind to an HRG polypeptide, HRG-depleted plasma and polynucleotides, vectors and host cells that encode HRG polypeptides (Patent Literature 3). In addition, there is a disclosure relating to the field of angiogenesis, and more particularly to the use of a substantially pure consecutive polypeptide having anti-angiogenic activity, including subfragments derived from a central region of HRG (Patent Literature 4). Further, there is also a disclosure of a depressant agent for neutrophil-vascular endothelial cell adhesion, containing HRG as an active ingredient (Patent Literature 5). As described above, actions of HRG are being elucidated, but there is no report of an antioxidant action thereof.

There is a disclosure that a protein of interest was generated using a gene expression cassette of a special structure (Patent Literature 6). In Patent Literature 6, there is a description that cells capable of stably and highly producing the protein of interest are obtained through use of the gene expression cassette, and as examples of the generated protein, HRG, an HRG-Fc fusion protein, and the like are described in Examples.

In phagocytes including neutrophils, reactive oxygen is produced through activation of NAD(P)H oxidase present in a cell membrane by bacteria, various particulate or soluble stimulants, and the like. This is reported in, for example, Non Patent Literature 2. In Non Patent Literature 2, various actions of HRG on neutrophils are described, but any action thereof against reactive oxygen produced by neutrophils is not described.

### Citation List

### Non Patent Literature

[NPL 1] Blood, Vol. 117, No. 7, 2093-2101 (2011)
[NPL 2] EBioMedicine, 9, 180-194 (2016), Available online 4 June 2016

### Patent Literature

[PTL 1] JP 2001-288078 A
[PTL 2] JP 2014-019660 A
[PTL 3] JP 2004-527242 A
[PTL 4] JP 2007-528710 A
[PTL 5] JP 5807937 B2 (WO 2013/183494 A1)
[PTL 6] WO 2017/061354 A1

### Summary of Invention

### Technical Problem

An object of the present invention is to provide a production inhibitor and/or scavenging promoter for reactive oxygen.

### Solution to Problem

There is provided a production inhibitor and/or scavenging promoter for reactive oxygen, containing as an active ingredient a peptide having a histidine-rich sequence. The inventors of the present application have made extensive investigations in order to achieve the above-mentioned object, and as a result, have found for the first time that the peptide having the histidine-rich sequence has production-inhibiting and scavenging-activities on hydroxy radicals and peroxy radicals. Thus, the inventors have completed the present invention.

That is, the present invention includes the following.
1. A production inhibitor and/or scavenging promoter for reactive oxygen, containing as an active ingredient a peptide having a histidine-rich sequence.
2. The production inhibitor and/or scavenging promoter for reactive oxygen according to the above-mentioned item 1, wherein the histidine-rich sequence is a partial amino acid sequence selected from those constituting the amino acid sequence set forth in SEQ ID NO: 1, and comprising at least five amino acids among which two or more amino acids are histidine.
3. The production inhibitor and/or scavenging promoter for reactive oxygen according to the above-mentioned item 1 or 2, wherein the histidine-rich sequence is the amino acid sequence identified by any one or a plurality of the following SEQ ID NOs: 2 to 8;
   (SEQ ID NO: 2) DLHPH
   (SEQ ID NO: 3) KHHSH
   (SEQ ID NO: 4) EQHPH
   (SEQ ID NO: 5) GHHPH
   (SEQ ID NO: 6) AHHPH
   (SEQ ID NO: 7) EHDTH
   (SEQ ID NO: 8) RQHPH.
4. The production inhibitor and/or scavenging promoter for reactive oxygen according to the above-mentioned item 2 or 3, wherein the peptide comprising the histidine-rich sequence, which is as the active ingredient, comprising 1 to 20 histidine-rich sequences.
5. The production inhibitor and/or scavenging promoter for reactive oxygen according to the above-mentioned item 1, wherein the peptide comprising the histidine-rich sequence, which is as the active ingredient, comprising at least one kind of amino acid sequence selected from the group consisting of: the amino acid sequence identified by amino acids 330 to 389 from the N-terminus of the amino acid sequence set forth in SEQ ID NO: 1; and the amino acid sequence comprising one or a plurality of amino acids substituted, deleted, added, or inserted in the amino acid sequence identified by amino acids 330 to 389 from the N-terminus of the amino acid sequence set forth in SEQ ID NO: 1.
6. The production inhibitor and/or scavenging promoter for reactive oxygen according to the above-mentioned item 1, wherein the peptide comprising the histidine-rich sequence, which is as the active ingredient, comprising at least one kind of amino acid sequence selected from the group consisting of: the amino acid sequence set forth in SEQ ID NO: 1; and the amino acid sequence comprising one or a plurality of amino acids substituted, deleted, added, or inserted in the amino acid sequence set forth in SEQ ID NO: 1.
7. The production inhibitor and/or scavenging promoter for reactive oxygen according to any one of the above-mentioned items 1 to 6, wherein the reactive oxygen is a hydroxy radical and/or a peroxy radical.
8. The production inhibitor and/or scavenging promoter for reactive oxygen according to any one of the above-mentioned items 1 to 7, wherein the production inhibitor and/or scavenging promoter for reactive oxygen is for inhibiting production of excess *in vivo* reactive oxygen and/or promoting scavenging thereof.

A. A production-inhibiting method and/or scavenging-promoting method for reactive oxygen, including using as an active ingredient a peptide having a histidine-rich sequence.
B. The production-inhibiting method and/or scavenging-promoting method for reactive oxygen according to the above-mentioned item A, wherein the histidine-rich sequence is a partial amino acid sequence selected from those constituting the amino acid sequence set forth in SEQ ID NO: 1, and comprising at least five amino acids among which two or more amino acids are histidine.
C. The production-inhibiting method and/or scavenging-promoting method for reactive oxygen according to the above-mentioned item A or B, wherein the histidine-rich sequence is the amino acid sequence identified by any one or a plurality of the following SEQ ID NOs: 2 to 8;
   (SEQ ID NO: 2) DLHPH
   (SEQ ID NO: 3) KHHSH
   (SEQ ID NO: 4) EQHPH
   (SEQ ID NO: 5) GHHPH
   (SEQ ID NO: 6) AHHPH
   (SEQ ID NO: 7) EHDTH
   (SEQ ID NO: 8) RQHPH.
D. The production-inhibiting method and/or scavenging-promoting method for reactive oxygen according to the above-mentioned item B or C, wherein the peptide comprising the histidine-rich sequence, which is as the active ingredient, comprising 1 to 20 histidine-rich sequences.
E. The production-inhibiting method and/or scavenging-promoting method for reactive oxygen according to the above-mentioned item A, wherein the peptide comprising the histidine-rich sequence, which is as the active ingredient, comprising at least one kind of amino acid sequence selected from the group consisting of: the amino acid sequence identified by amino acids 330 to 389 from the N-terminus of the amino acid sequence set forth in SEQ ID NO: 1; and the amino acid sequence comprising one or a plurality of amino acids substituted, deleted, added, or inserted in the amino acid sequence identified by amino acids 330 to 389 from the N-terminus of the amino acid sequence set forth in SEQ ID NO: 1.
F. The production-inhibiting method and/or scavenging-promoting method for reactive oxygen according to the above-mentioned item A, wherein the peptide comprising the histidine-rich sequence, which is as the active ingredient, comprising at least one kind of amino acid sequence selected from the group consisting of: the amino acid sequence set forth in SEQ ID NO: 1; and the amino acid sequence comprising one or a plurality of amino acids substituted, deleted, added, or inserted in the amino acid sequence set forth in SEQ ID NO: 1.
G. The production-inhibiting method and/or scavenging-promoting method for reactive oxygen according to any one of the above-mentioned items A to F, wherein the reactive oxygen is a hydroxy radical and/or a peroxy radical.
H. The production-inhibiting method and/or scavenging-promoting method for reactive oxygen according to any one of the above-mentioned items A to G, wherein the production-inhibiting method and/or scavenging-promoting method for reactive oxygen is for inhibiting production of excess *in vivo* reactive oxygen and/or promoting scavenging thereof.

### Advantageous Effects of Invention

The production inhibitor and/or scavenging promoter for reactive oxygen containing as the active ingredient the peptide having the histidine-rich sequence of the present invention has production-inhibiting and scavenging activities on hydroxy radicals and peroxy radicals even in a cell-free system. The peptide having the histidine-rich sequence binds to divalent iron ions to inhibit the production of hydroxy radicals having extremely high reactivity in Fenton's reaction.

### Brief Description of Drawings

FIG. 1 is a diagram for illustrating the structures of HRG and a peptide having a histidine-rich sequence.
FIGS. 2 are graphs showing the antioxidant action of HRG against hydroxy radicals. FIG. 2A is a graph showing the antioxidant action of gallic acid (GA) serving as a control against hydroxy radicals. FIG. 2B is a graph showing the antioxidant actions of HRG at various concentrations against hydroxy radicals. FIG. 2C is a graph showing the antioxidant actions of HRG or GA at various concentrations against hydroxy radicals. (Example 1)
FIGS. 3 are graphs showing the antioxidant action of the histidine-rich peptide against hydroxy radicals. FIG. 3A is a graph showing the antioxidant actions of a buffer, HRG, the histidine-rich peptide, and a control peptide against hydroxy radicals. FIG. 3B is a graph showing the antioxidant actions of the histidine-rich peptide at various concentrations. FIG. 3C is a graph showing the antioxidant action of HRG or the histidine-rich peptide against hydroxy radicals. (Example 2)
FIG. 4-1 includes graphs showing the binding properties of HRG to various metal ions. The graphs show the binding properties of HRG to divalent iron ions, trivalent iron ions, and divalent cobalt ions. (Example 3)
FIG. 4-2 includes graphs showing the binding properties of HRG to various metal ions. The graphs show the binding properties of HRG to divalent zinc ions, divalent copper ions, and divalent nickel ions. (Example 3)
FIG. 5-1 includes graphs showing the binding properties of the histidine-rich peptide set forth in SEQ ID NO: 10 to various metal ions. The graphs show the binding properties of the histidine-rich peptide to divalent iron ions, trivalent iron ions, and divalent cobalt ions. (Example 3)
FIG. 5-2 includes graphs showing the binding properties of the histidine-rich peptide to various metal ions. The graphs show the binding properties of the histidine-rich peptide to divalent zinc ions, divalent copper ions, and divalent nickel ions. (Example 3)
FIG. 6-1 includes graphs showing the binding properties of a control peptide set forth in SEQ ID NO: 11 to various metal ions. The graphs show the binding properties of the control peptide to divalent iron ions, trivalent iron ions, and divalent cobalt ions. (Example 3)
FIG. 6-2 includes graphs showing the binding properties of the control peptide to various metal ions. The graphs show the binding properties of the control peptide to divalent zinc ions, divalent copper ions, and divalent nickel ions. (Example 3)
FIGS. 7 are graphs showing antioxidant actions against peroxy radicals. FIG. 7A is a graph showing the antioxidant action of Trolox (Tro) serving as a control against peroxy radicals. FIG. 7B is a graph showing the antioxidant actions of HRG at various concentrations against peroxy radicals. FIG. 7C is a graph showing the antioxidant actions of HRG or Trolox at various concentrations against peroxy radicals. (Example 4)
FIGS. 8 are graphs showing the antioxidant action of the histidine-rich peptide against peroxy radicals. FIG. 8A is a graph showing the antioxidant actions of a buffer, HRG, the histidine-rich peptide, and the control peptide against peroxy radicals. FIG. 8B is a graph showing the antioxidant actions of the histidine-rich peptide at various concentrations against peroxy radicals. FIG. 8C is a graph showing the antioxidant action of HRG or the histidine-rich peptide against peroxy radicals. (Example 5)
FIGS. 9 are graphs showing results in which HRG has no antioxidant action against superoxide. (Reference Example 1)
FIGS. 10 are graphs showing results in which HRG has no catalase-like activity. (Reference Example 2)

### Description of Embodiments

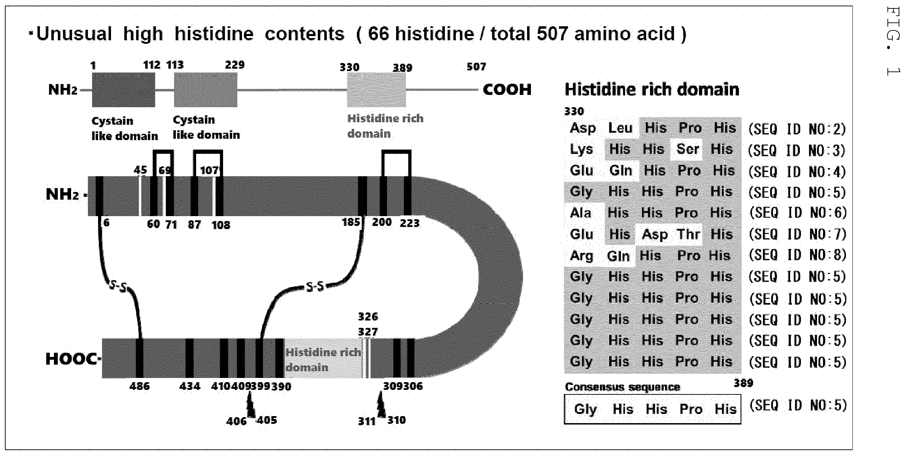

The present invention relates to "a production inhibitor and/or scavenging promoter for reactive oxygen, containing as an active ingredient a peptide having a histidine-rich sequence." Mature HRG is the histidine-rich protein having the amino acid sequence set forth in SEQ ID NO: 1 and formed of a total of 507 amino acids including 66 histidines. In the amino acid sequence forming mature HRG, the region identified by amino acids 330 to 389 from the N-terminus is called a histidine-rich domain (see FIG. 1) .

The "peptide having the histidine-rich sequence" serving as the active ingredient contained in the production inhibitor and/or scavenging promoter for reactive oxygen of the present invention includes a mature HRG protein having the amino acid sequence set forth in SEQ ID NO: 1, and also includes a peptide, polypeptide, or protein that is a part selected from those constituting the amino acid sequence set forth in SEQ ID NO: 1, and that contains the histidine-rich domain at one or a plurality of sites. Further, the "peptide having the histidine-rich sequence" of the present invention only needs to be a peptide, polypeptide, or protein containing the "histidine-rich sequence" to be described later, and is not limited to the peptide, polypeptide, or protein having the amino acid sequence set forth in SEQ ID NO: 1 or a partial sequence selected from those constituting the amino acid sequence set forth in SEQ ID NO: 1. It is suitable that the peptide be a part selected from those constituting the amino acid sequence set forth in SEQ ID NO: 1, and contain the amino acid sequence forming the histidine-rich domain. The concept of the "peptide having the histidine-rich sequence" serving as the active ingredient hereinafter encompasses HRG as well.

Amino Acid Sequence of Mature HRG (SEQ ID NO: 1)

VSPTDCSAVEPEAEKALDLINKRRRDGYLFQLLRIADAHLDRVENTTVYYLVLDVQESDCSV

LSRKYWNDCEPPDSRRPSEIVIGQCKVIATRHSHESQDLRVIDFNCTTSSVSSALANTKDSP

VLIDFFEDTERYRKQANKALEKYKEENDDFASFRVDRIERVARVRGGEGTGYFVDFSVRNCP

RHHFPRHPNVFGFCRADLFYDVEALDLESPKNLVINCEVFDPQEHENINGVPPHLGHPFHWG

GHERSSTTKPPFKPHGSRDHHHPHKPHEHGPPPPPDERDHSHGPPLPQGPPPLLPMSCSSCQ

HATFGTNGAQRHSHNNNSSDLHPHKHHSHEQHPHGHHPHAHHPHEHDTHRQHPHGHHPHGHH

PHGHHPHGHHPHGHHPHCHDFQDYGPCDPPPHNQGHCCHGHGPPPGHLRRRGPGKGPRPFHC

RQIGSVYRLPPLRKGEVLPLPEANFPSFPLPHHKHPLKPDNQPFPQSVSESCPGKFKSGFPQ

VSMFFTHTFPK

Herein, the "histidine-rich sequence" in the "peptide having the histidine-rich sequence" refers to a sequence that is a part selected from those constituting the amino acid sequence set forth in SEQ ID NO: 1, and that contains at least five amino acids among which two or more, preferably three or more amino acids are histidine. Specific examples thereof include amino acid sequences set forth in SEQ ID NOS: 2 to 8.

Histidine-rich Sequence
(SEQ ID NO: 2) DLHPH
(SEQ ID NO: 3) KHHSH
(SEQ ID NO: 4) EQHPH
(SEQ ID NO: 5) GHHPH
(SEQ ID NO: 6) AHHPH
(SEQ ID NO: 7) EHDTH
(SEQ ID NO: 8) RQHPH

The "peptide having the histidine-rich sequence" serving as the active ingredient includes a peptide having the histidine-rich sequence, for example, the histidine-rich sequence set forth in any one of SEQ ID NOs: 2 to 8 itself, or includes a peptide containing, for example, two or more histidine-rich sequences each set forth in any one of SEQ ID NOs: 2 to 8. In the case of containing two or more histidine-rich sequences each set forth in any one of SEQ ID NOs: 2 to 8, the peptide includes two or more of the same histidine-rich sequence, or the peptide includes histidine-rich sequences different from each other. The number of "histidine-rich sequences" contained in the peptide having the histidine-rich sequence is not particularly limited but 1 or a plurality, and is, for example, from 1 to 20 or from 1 to 15, preferably from 1 to 12, and more preferably from 2 to 12. In the case of containing the histidine-rich sequences at a plurality of sites, the sequences may optionally be contained consecutively, or the different amino acid sequence may optionally be interposed therebetween. The amino acid sequence set forth in SEQ ID NO: 1 contains one histidine-rich sequence identified by SEQ ID NO: 2, one histidine-rich sequence identified by SEQ ID NO: 3, one histidine-rich sequence identified by SEQ ID NO: 4, six histidine-rich sequences each identified by SEQ ID NO: 5, one histidine-rich sequence identified by SEQ ID NO: 6, one histidine-rich sequence identified by SEQ ID NO: 7, and one histidine-rich sequence identified by SEQ ID NO: 8.

The "peptide having the histidine-rich sequence" serving as the active ingredient may optionally be the peptide or protein containing at least one kind of amino acid sequence selected from the group consisting of: the amino acid sequence identified by amino acids 330 to 389 from the N-terminus of the amino acid sequence set forth in SEQ ID NO: 1; and the amino acid sequence having one or a plurality of amino acids substituted, deleted, added, or inserted in the amino acid sequence identified by amino acids 330 to 389 from the N-terminus of the amino acid sequence set forth in SEQ ID NO: 1. Further, the "peptide having the histidine-rich sequence or the histidine-rich glycoprotein" serving as the active ingredient may optionally be a peptide or protein containing at least one kind of amino acid sequence selected from the group consisting of: the amino acid sequence set forth in SEQ ID NO: 1; and the amino acid sequence having one or a plurality of amino acids substituted, deleted, added, or inserted in the amino acid sequence set forth in SEQ ID NO: 1.

When the peptide having the histidine-rich sequence serving as the active ingredient of the present invention is HRG, the mature HRG may optionally be generated by: a method of preparing the mature HRG through isolation/purification from a biological component; a method of generating the mature HRG through use of a recombination technology; or synthesis. The isolation/purification from a biological component can be, for example, purification and/or isolation from a biological component such as: blood, for example, plasma or serum; spinal fluid; or lymphatic fluid. The biological component is suitably a blood component, such as plasma or serum. A method known *per se* or any method to be developed in the future may be applied as a method for the isolation/purification from a biological component. For example, the mature HRG may optionally be prepared by passing plasma through an affinity column prepared using a nickel-nitrilotriacetic acid (Ni-NTA) agarose resin.

When the peptide having the histidine-rich sequence serving as the active ingredient of the present invention is to be generated using a recombination technology, a method known *per se* or any method to be developed in the future may be applied. The peptide having the histidine-rich sequence may optionally be prepared by cloning cDNA encoding the peptide into an expression vector. For example, the peptide can be a protein biosynthesized from the whole or part of the nucleotides identified by GenBank Accession No. NM000412. For example, the peptide may also be optionally generated by cloning full-length cDNA encoding the amino acid sequence (SEQ ID NO: 1) of mature HRG, or cDNA encoding part thereof, into an expression vector. Specifically, a method described in Patent Literature 6 may be applied as a method of generating HRG through use of a recombination technology. For example, a full-length cDNA encoding HRG, or cDNA encoding a part having the activity of HRG, such as a full-length cDNA encoding the amino acid sequence (SEQ ID NO: 1) of mature HRG, or cDNA encoding part thereof, is cloned into an expression vector to prepare an HRG expression vector.

The peptide having the histidine-rich sequence may optionally be generated by a peptide generation method known *per se.* For example, the peptide can be generated by a peptide synthesis method.

The kind of the reactive oxygen to be subjected to production inhibition and/or scavenging promotion by the "production inhibitor and/or scavenging promoter for reactive oxygen" of the present invention is not particularly limited, but a hydroxy radical or a peroxy radical is particularly suitable. Reactive oxygen is a generic term for more reactive compounds into which oxygen molecules contained in the atmosphere are converted. Reactive oxygen is produced in the following order through the capture of unpaired electrons by an oxygen molecule: superoxide, hydrogen peroxide, a hydroxy radical, and a peroxy radical. Superoxide is the first reduced form to be produced from an oxygen molecule, is a precursor of the other reactive oxygen species, and reacts with nitric oxide, which has a role important for a living body, to eliminate its action. Reactive oxygen serves to eliminate a toxin or the like that has entered a living body or has been produced therein, and can eliminate a toxin function by oxidizing the toxin with its oxidizing power. However, excessive production of reactive oxygen, or production thereof in a site that should be free thereof leads to the disturbance of an equilibrium relationship between production and scavenging in that local site, resulting in so-called oxidative stress-loaded conditions, and reactive oxygen attacks constituent biomolecules of membranes and tissues of a living body to induce various diseases . That is, oxidative stress destroys not only the toxin but also cells all over the body, and thus serves as a cause of aging or causes various diseases. Excess reactive oxygen not only inflicts various kinds of damage on biological membranes, nucleic acids, proteins, biologically active factors, and the like, causing membrane lipid peroxidation reaction, oxidative damage, protein denaturation, and the like, but also acts as a regulatory factor for signal transduction in many life phenomena.

As environmental factors for the production of reactive oxygen, there are given air pollutants, radiations, certain kinds of pharmaceutical agents, ultraviolet light, tobacco/cigarette, and the like, and as *in vivo* factors, there are given certain kinds of enzymes in the mitochondrial electron transport system and the microsomal electron transport system, oxidase-based enzymes, iron-containing protein, and the like. In relation to inflammation, ischemic diseases, and the like, phagocytes including neutrophils also attract attention. In phagocytes including neutrophils, reactive oxygen is produced through activation of NAD(P)H oxidase present in a cell membrane bybacteria, various particulate or soluble stimulants, and the like. *In vivo* reactive oxygen species have been elucidated as causes of various kinds of diseases and poor health, and reactive oxygen species are said to be one of the causes of various kinds of cell death, cancer, arteriosclerosis, hypertension, diabetes, and the like. It has also been made clear that excess reactive oxygen species produced by neutrophils act in a tissue-damaging manner in inflammatory reactions including ischemia-reperfusion injury and autoimmune diseases. Generated superoxide is enzymatically or non-enzymatically reduced into a reactive oxygen species having higher reactivity (hydroxy radical or peroxy radical) . It is said that reactive oxygen species produced by activated neutrophils are involved in the pathogenesis of acute respiratory distress syndrome, ulcerative colitis, acute gastric mucosal lesion, Behçet's disease, and the like.

The "production inhibitor and/or scavenging promoter for reactive oxygen" of the present invention may optionally contain an appropriate amount of a pharmacologically acceptable carrier or the like in addition to an effective amount of the peptide having the histidine-rich sequence serving as the active ingredient. Examples of the pharmacologically acceptable carrier may optionally include excipients (including monosaccharides, oligosaccharides, and polysaccharides), disintegrants or disintegration aids, binders, lubricating agents, lubricants, coating agents, dyes, diluents, bases, solubilizing agents or dissolution aids, tonicity agents, pH adjusters, stabilizers, propellants, thickeners (including chitin, chitosan, and polysaccharide thickeners), and pressure-sensitive adhesives.

The "production inhibitor and/or scavenging promoter for reactive oxygen" of the present invention can be used as a therapeutic agent or therapeutic adjunct for various kinds of diseases and poor health resulting from *in vivo* reactive oxygen species. Specific examples of the various kinds of diseases and poor health include various kinds of cell death, cancer, arteriosclerosis, hypertension, diabetes, ischemia-reperfusion injury, acute respiratory distress syndrome, acute gastric mucosal lesion, and an autoimmune disease. Examples of the autoimmune disease include ulcerative colitis and Behçet's disease.

The "production inhibitor and/or scavenging promoter for reactive oxygen" of the present invention can be locally or systemically administered to be used as the therapeutic agent or therapeutic adjunct for the diseases described above. The dose, administration frequency, and administration period thereof only need to be appropriately set in consideration of the kind and symptoms of a target disease, and the age and sex of a subject. When the "production inhibitor and/or scavenging promoter for reactive oxygen" of the present invention is in any of forms for parenteral administration, the forms may optionally include sterilized, aqueous or nonaqueous solutions, suspensions, and emulsions. Examples of nonaqueous diluents are propylene glycol, polyethylene glycol, a plant oil, such as olive oil, and an organic ester composition, such as ethyl oleate, which are suitable for injection. Aqueous carriers may optionally include pharmacologically acceptable water, alcoholic/aqueous solutions, emulsions, suspensions, saline, and buffered media. Parenteral carriers may include sodium chloride solution, Ringer's dextrose, dextrose and sodium chloride, lactated Ringer's, and fixed oils. Intravenous carriers may include, for example, fluid replenishers, and nutrient and electrolyte replenishers (such as those based on Ringer's dextrose). The "production inhibitor and/or scavenging promoter for reactive oxygen" of the present invention may optionally further contain one kind or two or more kinds of a preservative, a stabilizer, and other additives, such as antimicrobial compounds (including an antibacterial agent and an antibiotic), an antioxidant, a chelating agent, and an inert gas.

When used as the therapeutic agent or therapeutic adjunct for the diseases described above, the "production inhibitor and/or scavenging promoter for reactive oxygen" of the present invention can be administered in combination with one kind or two or more kinds of other pharmaceutical formulations. The other pharmaceutical formulations that may optionally be used in combination are not particularly limited, and may each generally be any already commercially available therapeutic agent or preventive agent, or any therapeutic agent or preventive agent to be developed in the future. The other pharmaceutical formulations are each suitably a pharmaceutical formulation having an action mechanism different from that of the "production inhibitor and/or scavenging promoter for reactive oxygen" of the present invention. Through combined use with the "production inhibitor and/or scavenging promoter for reactive oxygen" of the present invention, it is also possible to reduce the doses of the other pharmaceutical formulations. When the other pharmaceutical formulations have side effects, the combined use thereof with the "production inhibitor and/or scavenging promoter for reactive oxygen" of the present invention can reduce the administration of the other pharmaceutical formulations, and hence is useful.

Besides the above-mentioned pharmaceutical, the "production inhibitor and/or scavenging promoter for reactive oxygen" of the present invention may also be used as: a raw material for a cosmetic, a health food, a food for specified health use, a functional food, or the like; a cosmetic supplement; or a food supplement.

### Examples

The present invention is specifically described by way of the following Examples, experimental examples, and comparative examples, but it goes without saying that the present invention is not limited by these Examples and the like.

### (Example 1) Antioxidant Action against Hydroxy Radicals 1

In this experimental example, HRG was investigated for its antioxidant activity against hydroxy radicals. HRG to be used in this Example and Examples and Reference Examples to be described later was generated by a method described in Example 1 of Patent Literature 5 (WO 2013/183494 A1) using human plasma as a starting material. HRG samples having various concentrations were generated through dilution with Hanks' solution (Hanks' Balanced Salt Solution: HBSS) . As a positive control, gallic acid (GA) was used.

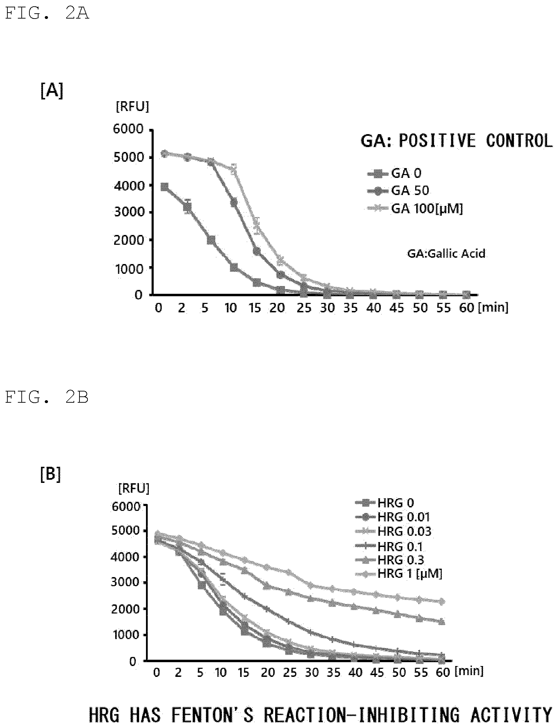

Antioxidant activity against hydroxy radicals was measured with OxiSelect™ HORAC Activity Assay Kit (Cell Biolabs, San Diego, CA) in accordance with the specifications . A fluorescent probe solution was added to a sample solution, the mixture was incubated at 37°C for 30 minutes, and a hydroxy radical initiator (H₂O₂) and Fenton's reagent were added, followed by the measurement of a fluorescence intensity (Ex/Em=485 nm/538 nm) based on the oxidation of the fluorescent probe by hydroxy radicals via hydrogen atom transfer (HAT). In the presence of hydroxy radicals, the fluorescence of the fluorescent probe is quenched, and the quenching is inhibited when antioxidant activity is present. As the sample solution, 0.01 µM to 1 µM HRG solutions, or 50 µM to 100 µM GA solutions were used.
A. For each of the 50 µM to 100 µM gallic acid (GA) sample solutions, their fluorescence intensities (Ex/Em=485 nm/538 nm) were measured every 5 minutes for 60 minutes in accordance with the above-mentioned measurement method. The fluorescence intensities of GA and a blank were plotted on the vertical axis, and the measurement times were plotted on the horizontal axis. An antioxidant action against hydroxy radicals was recognized in a manner dependent on the concentration of GA (FIG. 2A).
B. For each of the 0 µM to 1 µM HRG sample solutions, their fluorescence intensities (Ex/Em=485 nm/538 nm) were measured every 5 minutes for 60 minutes in accordance with the above-mentioned measurement method. The fluorescence intensities of HRG and a blank were plotted on the vertical axis, and the measurement times were plotted on the horizontal axis. An antioxidant action against hydroxy radicals was recognized in a manner dependent on the concentration of HRG (FIG. 2B).
C. Antioxidant Activity against Hydroxy Radicals (NetAUC)

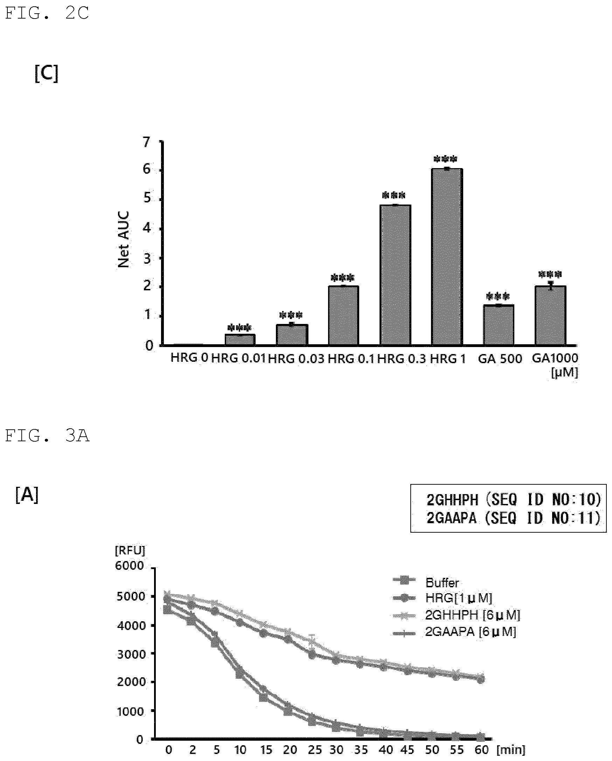

The difference between the area under the curve of the fluorescence intensity of GA or HRG (AUCₛₐₘₚₗₑ) and the area under the curve of the blank (AUC_{blank}) was measured every 5 minutes for 60 minutes after the initiation of reaction, and NetAUC was calculated. It was recognized that HRG had a potent antioxidant action against hydroxy radicals (FIG. 2C).

### (Example 2) Antioxidant Action against Hydroxy Radicals 2

In this Example, the histidine-rich peptide (SEQ ID NO: 10) was investigated for its antioxidant action against hydroxy radicals by the same technique as that of Example 1. A control peptide (SEQ ID NO: 11) was similarly investigated.
- Histidine-rich peptide: GHHPHGHHPH (SEQ ID NO: 10)
- Control peptide: GAAPAGAAPA (SEQ ID NO: 11)

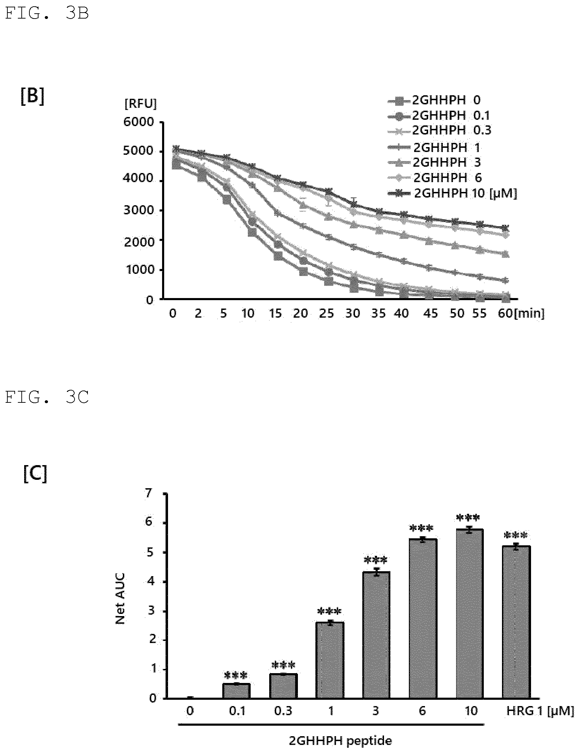

The amino acid sequence set forth in SEQ ID NO: 10 is a sequence obtained by linking two amino acid sequences each set forth in SEQ ID NO: 5 to each other, and the amino acid sequence set forth in SEQ ID NO: 11 is a sequence obtained by substituting histidine in the amino acid sequence set forth in SEQ ID NO: 10 with arginine. Each of the peptides was generated through utilization of the custom peptide synthesis service of Sigma-Genosys. In addition, the histidine-rich peptide and the control peptide of this Example were also used in Examples and Reference Examples as described later.
A. The fluorescent probe solution was added to each solution of HRG (1 µM), the histidine-rich peptide (6 µM), or the control peptide (6 µM), and antioxidant activity against hydroxy radicals was measured by the same technique as that of Example 1. HRG and the histidine-rich peptide showed potent antioxidant actions, whereas the control peptide showed no antioxidant action as with a buffer (FIG. 3A).
B. The fluorescent probe solution was added to histidine-rich peptide solutions (0.1 µM to 10 µM), and the resultant mixtures were measured for their antioxidant activity against hydroxy radicals by the same technique as that of Example 1. The histidine-rich peptide showed potent antioxidant activity in a concentration-dependent manner (FIG. 3B).
C. Antioxidant Activity against Hydroxy Radicals (NetAUC)

The difference between the area under the curve of the fluorescence intensity of the histidine-rich peptide or HRG (AUCₛₐₘₚₗₑ) and the area under the curve of the blank (AUC_{blank}) was measured every 5 minutes for 60 minutes after the initiation of reaction to calculate NetAUC. It was recognized that the histidine-rich peptide and HRG each had a potent antioxidant action against hydroxy radicals (FIG. 3C).

### (Example 3) Metal Ion-binding Property

In this Example, in order to investigate the binding properties of HRG to metal ions serving as essential factors in Fenton's reaction, an experiment was performed using calorimetry. MicroCal iTC200 was used as a calorimeter, and binding properties between various metals (iron, copper, cobalt, zinc, and nickel) and samples were measured in accordance with the specifications.

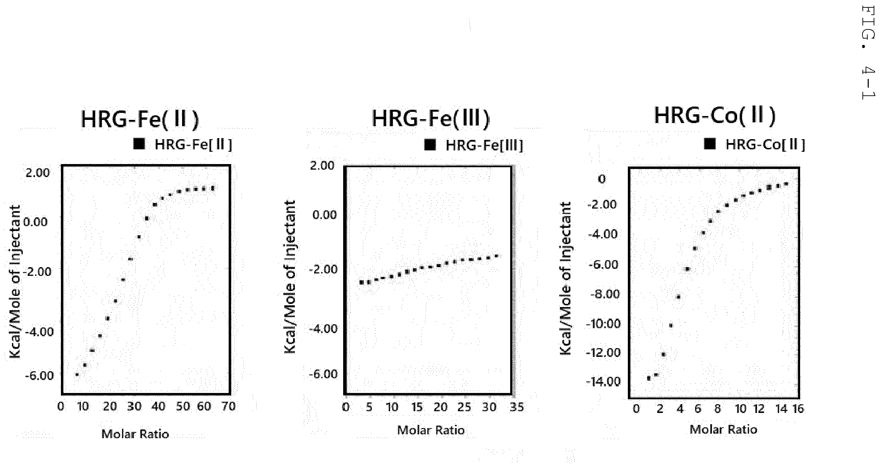

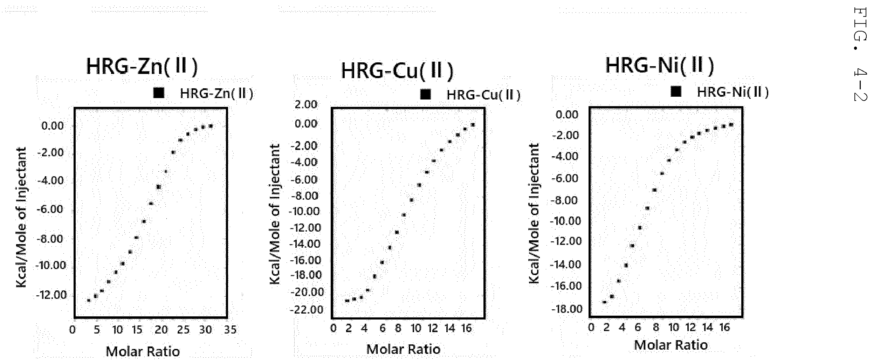

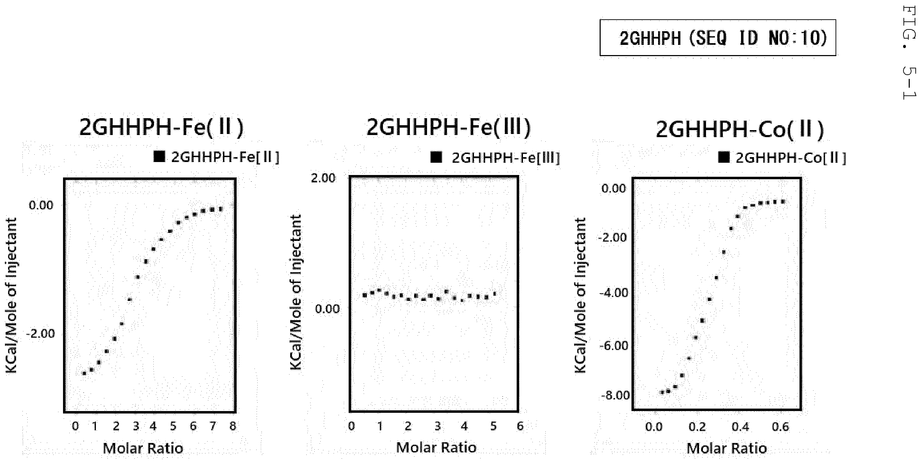

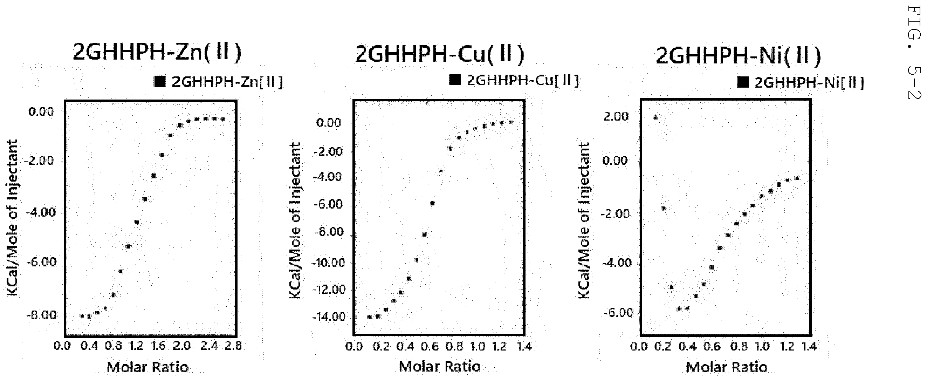

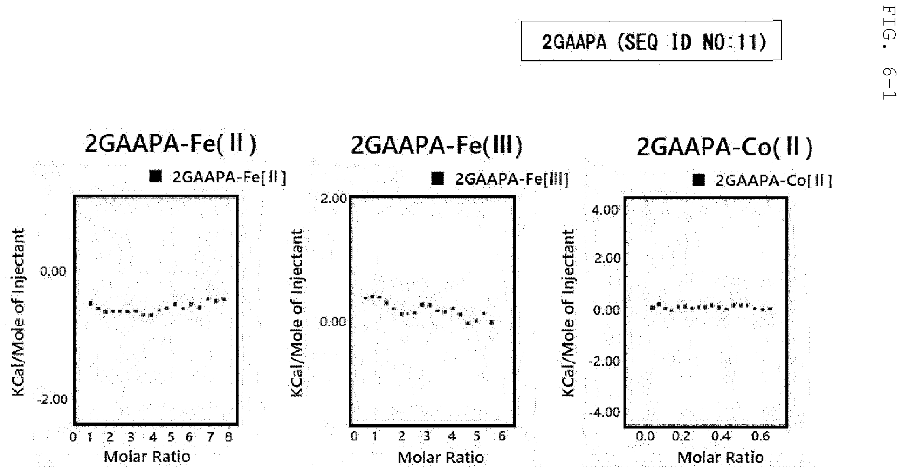

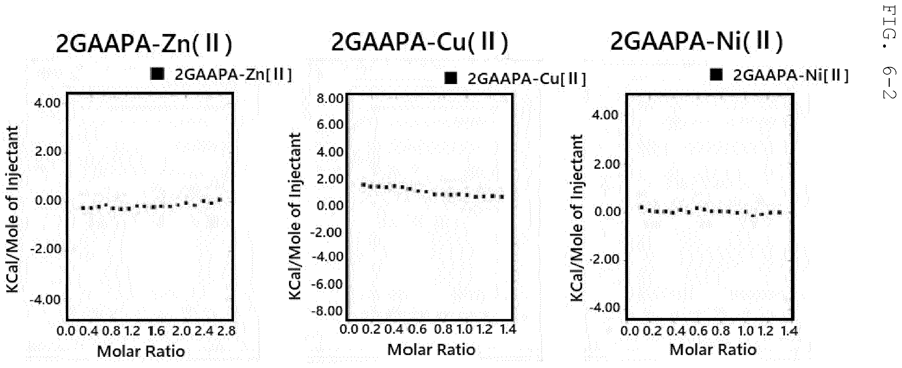

As a result, it was recognized that HRG bound to divalent metal ions (iron, copper, cobalt, zinc, and nickel) (FIG. 4-1 and FIG. 4-2), but did not bind to trivalent iron ions (FIG. 4-1). This was the first finding that HRG bind to divalent iron ions. Further, it was revealed that a large number of divalent metal ions bind to one molecule of HRG, revealing that HRG efficiently bind to divalent metal ions (FIG. 4-1). Further, it was recognized that the histidine-rich peptide also bind to divalent metal ions (iron, copper, cobalt, zinc, and nickel), but does not bind to trivalent iron ions (FIG. 5-1 and FIG. 5-2). The control peptide did not bind to any of the various metal ions (FIG. 6-1 and FIG. 6-2). It was recognized from those results that histidine was important for the binding to divalent metal ions.

Further, it was revealed that a large number of divalent metal ions bind to one molecule of HRG, revealing that HRG efficiently bind to divalent metal ions. Further, the histidine-rich peptide (see SEQ ID NO: 10) was the key of the binding, and the control peptide (see SEQ ID NO: 11) did not bind to divalent metal ions. Accordingly, it was shown that histidine was important for the binding. Thus, it was revealed that HRG bind to divalent metal ions (iron, cobalt, and the like) essential to Fenton's reaction serving as a hydroxy radical production pathway, to thereby inhibit the reaction.

### (Example 4) Antioxidant Action against Peroxy Radicals 1

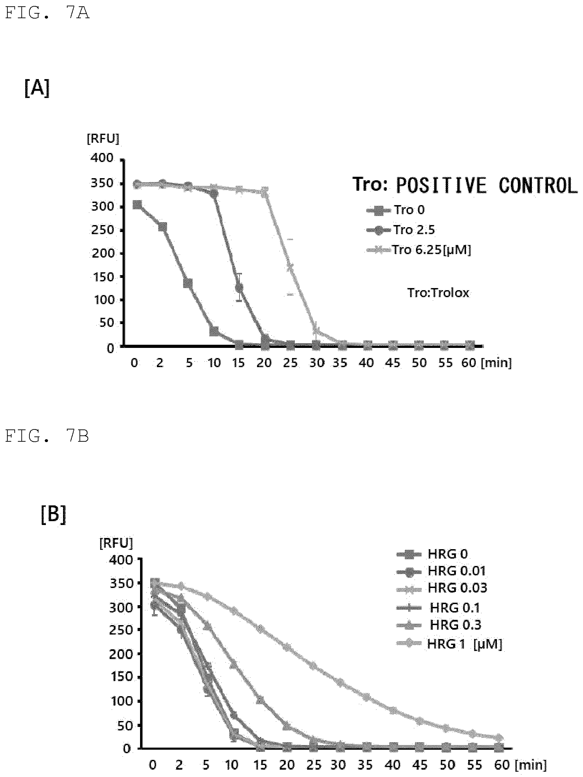

In this Example, HRG was investigated for its antioxidant action against peroxy radicals. As a positive control, Trolox was used. Antioxidant activity against peroxy radicals was measured with OxiSelect™ ORAC Activity Assay Kit (Cell Biolabs, San Diego, CA) in accordance with the specifications. The fluorescent probe solution was added to a sample solution, the mixture was incubated at 37°C for 30 minutes, and added with 2,2'-azobis-(2-methylpropionamidine) dihydrochloride (AAPH), followed by immediate measurement of fluorescence intensity (Ex/Em=485 nm/538 nm). In the presence of peroxy radicals, the fluorescence of the fluorescent probe is quenched, and the quenching is inhibited when antioxidant activity is present.
A. For each of 0 µM to 6.25 µM Trolox sample solutions, their fluorescence intensities (Ex/Em=485 nm/538 nm) were measured every 5 minutes for 60 minutes in accordance with the above-mentioned measurement method. An antioxidant action against peroxy radicals was recognized in a manner dependent on the concentration of Trolox (FIG. 7A).
B. For each of 0 µM to 1 µM HRG sample solutions, their fluorescence intensity (Ex/Em=485 nm/538 nm) were measured every 5 minutes for 60 minutes in accordance with the above-mentioned measurement method. An antioxidant action against peroxy radicals was recognized in a manner dependent on the concentration of HRG (FIG. 7B).
C. Antioxidant Activity against Peroxy Radicals (NetAUC)

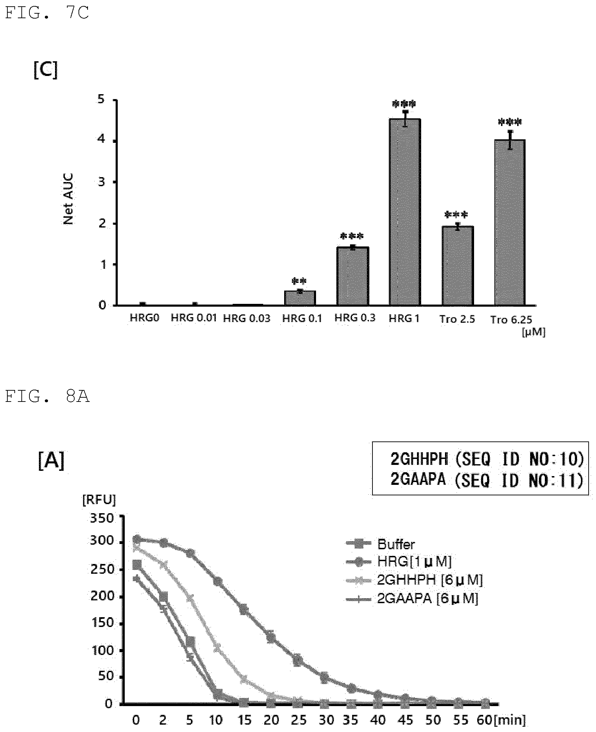

The difference between the area under the curve of the fluorescence intensity of Trolox or HRG (AUCₛₐₘₚₗₑ) and the area under the curve of the blank (AUC_{blank}) was measured every 5 minutes for 60 minutes after the initiation of reaction to calculate NetAUC. It was recognized that HRG has an antioxidant action against peroxy radicals (FIG. 7C).

### (Example 5) Antioxidant Action against Peroxy Radicals 2

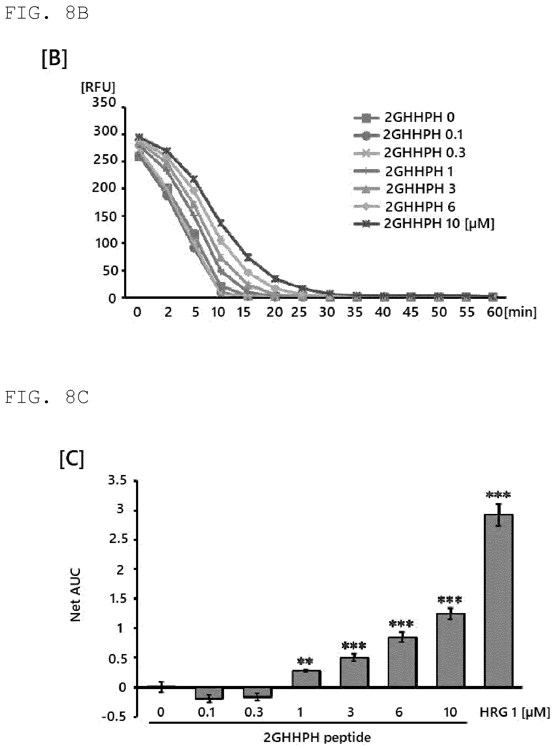

In this Example, the histidine-rich peptide was investigated for its antioxidant action against peroxy radicals in the same manner as in Example 4.
A. The fluorescent probe solution was added to each solution of HRG (1 µM), the histidine-rich peptide (6 µM), or the control peptide (6 µM), and antioxidant activity against peroxy radicals was measured by the same technique as that of Example 4. HRG showed a potent antioxidant action, whereas the histidine-rich peptide showed an antioxidant action, which was slightly weaker. The control peptide showed no antioxidant action as with a buffer (FIG. 8A).
B. The fluorescent probe solution was added to histidine-rich peptide solutions (0.1 µM to 10 µM), and antioxidant activity against peroxy radicals was measured by the same technique as that of Example 4. The histidine-rich peptide showed antioxidant activity in a slightly concentration-dependent manner (FIG. 8B).
C. Antioxidant Activity against Peroxy Radicals (NetAUC)

The difference between the area under the curve of the fluorescence intensity of the histidine-rich peptide or HRG (AUCₛₐₘₚₗₑ) and the area under the curve of the blank (AUC_{blank}) was measured every 5 minutes for 60 minutes after the initiation of reaction to calculate NetAUC. HRG had an antioxidant action against peroxy radicals, but the antioxidant activity of the histidine-rich peptide was weak as compared to HRG (FIG. 8C). It was suggested that the effect of HRG against peroxy radicals was not an action based only on its histidine-rich domain.

### (Reference Example 1) Antioxidant Action of HRG against Superoxide

In this Reference Example, the antioxidant action of HRG against superoxide was investigated through use of DetectX Superoxide Dismutase (SOD) Colorimetric Activity Kit (Arbor Assays, Ann Arbor, MI). HRG used was plasma-derived HRG generated by the method described in Example 1. Superoxide anion (O²⁻) is generated by a xanthine/xanthine oxidase (XOD) system, and is detected through use of a chromogen solution. Superoxide dismutase (SOD) is a metalloenzyme that catalyzes the conversion of O²⁻ into an oxygen molecule and hydrogen peroxide. The presence of SOD decreases the O²⁻ concentration to decrease a dye signal.

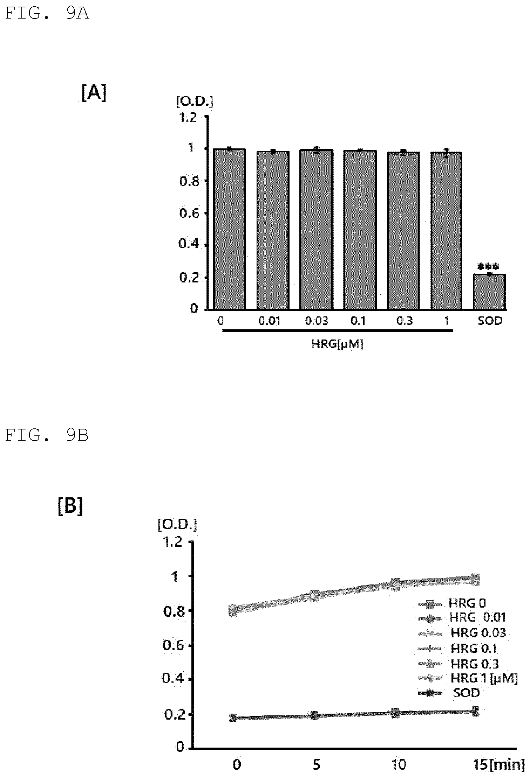

In accordance with the specifications, xanthine oxidase, and a substrate were added to 0.01 µM to 1 µM HRG solutions, and the mixtures were incubated at room temperature for 20 minutes. After that, the reaction solutions were each measured for its absorbance at a wavelength of 450 nm. As a control, measurement was similarly performed for SOD. As a result, it was revealed that HRG had no scavenging activity against superoxide (FIG. 9).

### (Reference Example 2) Catalase Action of HRG

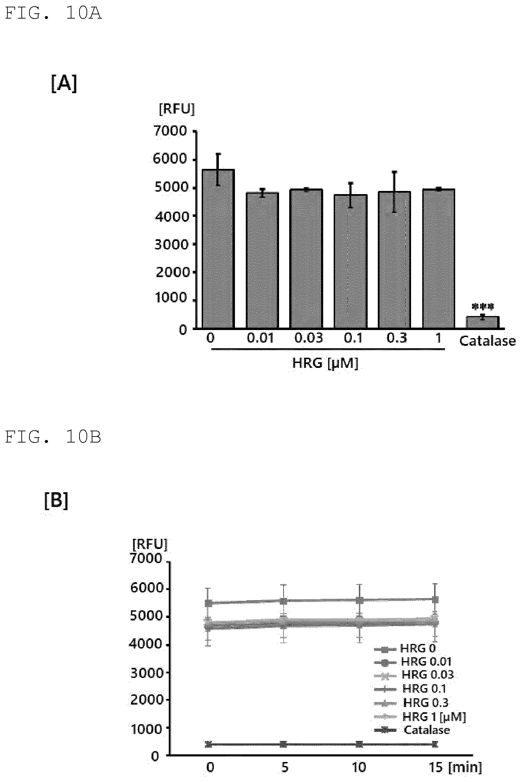

In this Reference Example, whether HRG had catalase-like activity was investigated through use of OxiSelect™ Catalase Activity Assay Kit (Cell Biolabs, San Diego, CA). HRG used was plasma-derived HRG generated by the method described in Example 1. A prescribed amount of hydrogen peroxide was allowed to react with 0.01 µM to 1 µM HRG. Next, the remaining hydrogen peroxide was incubated in the presence of HRP with a probe (colorimetric measurement: DHBS+AAP; and fluorescence measurement: ADHP (10-Acetyl-3,7-dihydroxyphenoxazine)) at 37°C for 30 minutes, and the resulting fluorescence intensity (Ex/Em=544 nm/590 nm) was measured. As a control, measurement was similarly performed for catalase. As a result, it was revealed that HRG had no catalase-like activity (FIG. 10).

It was recognized from the above-mentioned results that HRG and the histidine-rich peptide each showed a potent antioxidant activity against hydroxy radicals. It was recognized that such a potent antioxidant activity was based on the action of a region having the histidine-rich sequence. HRG also showed an antioxidant activity against peroxy radicals. Meanwhile, HRG hardly showed an antioxidant action against superoxide.

### Industrial Applicability

As described in detail above, it was recognized that the peptide having the histidine-rich sequence of the present invention shows a potent antioxidant activity against hydroxy radicals; and such a potent antioxidant activity is based on the action of the histidine-rich domain of HRG or a region having the histidine-rich sequence. HRG also showed an antioxidant activity against peroxy radicals. Meanwhile, HRG has hardly showed an antioxidant action against the superoxide.

Although various attempts have been made to develop antioxidants against reactive oxygen, the hydroxy radical has an extremely high reactivity and an extremely short lifetime, and hence the development of a pharmaceutical agent that effectively acts on hydroxy radicals has not been developed. The peptide having the histidine-rich sequence of the present invention effectively inhibits the production of hydroxy radicals.

## Claims

1. A production inhibitor and/or scavenging promoter for reactive oxygen, comprising as an active ingredient a peptide having a histidine-rich sequence.

2. The production inhibitor and/or scavenging promoter for reactive oxygen according to claim 1, wherein the histidine-rich sequence is a partial amino acid sequence selected from those constituting the amino acid sequence set forth in SEQ ID NO: 1, and comprising at least five amino acids among which two or more amino acids are histidine.

3. The production inhibitor and/or scavenging promoter for reactive oxygen according to claim 1 or 2, wherein the histidine-rich sequence is the amino acid sequence identified by any one or a plurality of the following SEQ ID NOs: 2 to 8;
(SEQ ID NO: 2) DLHPH
(SEQ ID NO: 3) KHHSH
(SEQ ID NO: 4) EQHPH
(SEQ ID NO: 5) GHHPH
(SEQ ID NO: 6) AHHPH
(SEQ ID NO: 7) EHDTH
(SEQ ID NO: 8) RQHPH.

4. The production inhibitor and/or scavenging promoter for reactive oxygen according to claim 2 or 3, wherein the peptide comprising the histidine-rich sequence, which is as the active ingredient, comprising 1 to 20 histidine-rich sequences.

5. The production inhibitor and/or scavenging promoter for reactive oxygen according to claim 1, wherein the peptide comprising the histidine-rich sequence, which is as the active ingredient, comprising at least one kind of amino acid sequence selected from the group consisting of : the amino acid sequence identified by amino acids 330 to 389 from the N-terminus of the amino acid sequence set forth in SEQ ID NO: 1; and the amino acid sequence comprising one or a plurality of amino acids substituted, deleted, added, or inserted in the amino acid sequence identified by amino acids 330 to 389 from the N-terminus of the amino acid sequence set forth in SEQ ID NO: 1.

6. The production inhibitor and/or scavenging promoter for reactive oxygen according to claim 1, wherein the peptide comprising the histidine-rich sequence, which is as the active ingredient, comprising at least one kind of amino acid sequence selected from the group consisting of: the amino acid sequence set forth in SEQ ID NO: 1; and the amino acid sequence comprising one or a plurality of amino acids substituted, deleted, added, or inserted in the amino acid sequence set forth in SEQ ID NO: 1.

7. The production inhibitor and/or scavenging promoter for reactive oxygen according to any one of claims 1 to 6, wherein the reactive oxygen is a hydroxy radical and/or a peroxy radical.

8. The production inhibitor and/or scavenging promoter for reactive oxygen according to any one of claims 1 to 7, wherein the production inhibitor and/or scavenging promoter for reactive oxygen is for inhibiting production of excess *in vivo* reactive oxygen and/or promoting scavenging thereof.


SEQUENCE LISTING

<110>  National University Corporation Okayama University

<120>  Production inhibitor of Reactive Oxygen Species and/or erasing
       accelerator

<130>  GP19-1007PCT

<150>  JP P2018-105652
<151>  2018-05-31

<160>  11

<170>  PatentIn version 3.5

<210>  1
<211>  507
<212>  PRT
<213>  Homo sapiens

<400>  1

Val Ser Pro Thr Asp Cys Ser Ala Val Glu Pro Glu Ala Glu Lys Ala
1               5                   10                  15

Leu Asp Leu Ile Asn Lys Arg Arg Arg Asp Gly Tyr Leu Phe Gln Leu
            20                  25                  30

Leu Arg Ile Ala Asp Ala His Leu Asp Arg Val Glu Asn Thr Thr Val
        35                  40                  45

Tyr Tyr Leu Val Leu Asp Val Gln Glu Ser Asp Cys Ser Val Leu Ser
    50                  55                  60

Arg Lys Tyr Trp Asn Asp Cys Glu Pro Pro Asp Ser Arg Arg Pro Ser
65                  70                  75                  80

Glu Ile Val Ile Gly Gln Cys Lys Val Ile Ala Thr Arg His Ser His
                85                  90                  95

Glu Ser Gln Asp Leu Arg Val Ile Asp Phe Asn Cys Thr Thr Ser Ser
            100                 105                 110

Val Ser Ser Ala Leu Ala Asn Thr Lys Asp Ser Pro Val Leu Ile Asp
        115                 120                 125

Phe Phe Glu Asp Thr Glu Arg Tyr Arg Lys Gln Ala Asn Lys Ala Leu
    130                 135                 140

Glu Lys Tyr Lys Glu Glu Asn Asp Asp Phe Ala Ser Phe Arg Val Asp
145                 150                 155                 160

Arg Ile Glu Arg Val Ala Arg Val Arg Gly Gly Glu Gly Thr Gly Tyr
                165                 170                 175

Phe Val Asp Phe Ser Val Arg Asn Cys Pro Arg His His Phe Pro Arg
            180                 185                 190

His Pro Asn Val Phe Gly Phe Cys Arg Ala Asp Leu Phe Tyr Asp Val
        195                 200                 205

Glu Ala Leu Asp Leu Glu Ser Pro Lys Asn Leu Val Ile Asn Cys Glu
    210                 215                 220

Val Phe Asp Pro Gln Glu His Glu Asn Ile Asn Gly Val Pro Pro His
225                 230                 235                 240

Leu Gly His Pro Phe His Trp Gly Gly His Glu Arg Ser Ser Thr Thr
                245                 250                 255

Lys Pro Pro Phe Lys Pro His Gly Ser Arg Asp His His His Pro His
            260                 265                 270

Lys Pro His Glu His Gly Pro Pro Pro Pro Pro Asp Glu Arg Asp His
        275                 280                 285

Ser His Gly Pro Pro Leu Pro Gln Gly Pro Pro Pro Leu Leu Pro Met
    290                 295                 300

Ser Cys Ser Ser Cys Gln His Ala Thr Phe Gly Thr Asn Gly Ala Gln
305                 310                 315                 320

Arg His Ser His Asn Asn Asn Ser Ser Asp Leu His Pro His Lys His
                325                 330                 335

His Ser His Glu Gln His Pro His Gly His His Pro His Ala His His
            340                 345                 350

Pro His Glu His Asp Thr His Arg Gln His Pro His Gly His His Pro
        355                 360                 365

His Gly His His Pro His Gly His His Pro His Gly His His Pro His
    370                 375                 380

Gly His His Pro His Cys His Asp Phe Gln Asp Tyr Gly Pro Cys Asp
385                 390                 395                 400

Pro Pro Pro His Asn Gln Gly His Cys Cys His Gly His Gly Pro Pro
                405                 410                 415

Pro Gly His Leu Arg Arg Arg Gly Pro Gly Lys Gly Pro Arg Pro Phe
            420                 425                 430

His Cys Arg Gln Ile Gly Ser Val Tyr Arg Leu Pro Pro Leu Arg Lys
        435                 440                 445

Gly Glu Val Leu Pro Leu Pro Glu Ala Asn Phe Pro Ser Phe Pro Leu
    450                 455                 460

Pro His His Lys His Pro Leu Lys Pro Asp Asn Gln Pro Phe Pro Gln
465                 470                 475                 480

Ser Val Ser Glu Ser Cys Pro Gly Lys Phe Lys Ser Gly Phe Pro Gln
                485                 490                 495

Val Ser Met Phe Phe Thr His Thr Phe Pro Lys
            500                 505

<210>  2
<211>  5
<212>  PRT
<213>  Homo sapiens

<400>  2

Asp Leu His Pro His
1               5

<210>  3
<211>  5
<212>  PRT
<213>  Homo sapiens

<400>  3

Lys His His Ser His
1               5

<210>  4
<211>  5
<212>  PRT
<213>  Homo sapiens

<400>  4

Glu Gln His Pro His
1               5

<210>  5
<211>  5
<212>  PRT
<213>  Homo sapiens

<400>  5

Gly His His Pro His
1               5


<210>  6
<211>  5
<212>  PRT
<213>  Homo sapiens

<400>  6

Ala His His Pro His
1               5


<210>  7
<211>  5
<212>  PRT
<213>  Homo sapiens

<400>  7

Glu His Asp Thr His
1               5


<210>  8
<211>  5
<212>  PRT
<213>  Homo sapiens

<400>  8

Arg Gln His Pro His
1               5


<210>  9
<211>  10
<212>  PRT
<213>  Homo sapiens

<400>  9

Gly Ala Ala Pro Ala Gly Ala Ala Pro Ala
1               5                   10


<210>  10
<211>  10
<212>  PRT
<213>  Homo sapiens

<400>  10

Gly His His Pro His Gly His His Pro His
1               5                   10


<210>  11
<211>  10

<212>  PRT
<213>  Artificial

<220>
<223>  Standard peptide

<400>  11

Gly Ala Ala Pro Ala Gly Ala Ala Pro Ala
1               5                   10